Europäisches Patentamt

European Patent Office

Office européen des brevets

(19)

(11) Publication number: **0 085 036**
**A1**

# (12) EUROPEAN PATENT APPLICATION

(21) Application number: **83870003.7**

(22) Date of filing: **14.01.83**

(51) Int. Cl.³: **A 61 K 37/36**

(30) Priority: **18.01.82 US 340154**

(43) Date of publication of application: **03.08.83**
**Bulletin 83/31**

(84) Designated Contracting States: **AT BE CH DE FR GB IT LI NL SE**

(71) Applicant: **MONSANTO COMPANY, Patent Department 800 North Lindbergh Boulevard, St. Louis, Missouri 63166 (US)**

(72) Inventor: **de Geeter, Melvin Joseph, 16241 Bent Tree Drive, Chesterfield Missouri 63017 (US)**
Inventor: **Lanza, Gregory Mark, 1943 Greenpoint Drive, Apt 302, Kirkwood Missouri 63122 (US)**

(74) Representative: **Lunt, John Cooper et al, Monsanto Europe S.A. Patent Department Avenue de Tervuren 270-272 Letter Box No 1, B-1150 Brussels (BE)**

(54) **Method for improved bovine milk production.**

(57) Increased production of milk from cows is afforded by administering to cows an effective amount of biosynthetic bovine growth hormone or analog thereof.

EP 0 085 036 A1

ACTORUM AG

-1-

## METHOD FOR IMPROVED BOVINE
## MILK PRODUCTION

Increased milk production of cows is a matter of considerable commercial importance.

Accordingly, various means have been used in the past to increase such production. Such means include administration of hormones, e.g., natural bovine growth hormone, prolactin, caseinate materials, e.g. sodium and calcium caseinates, thyrotropin releasing hormone, thyroid stimulating hormone, thyroxin, triiodothyronine, estrogen and prostaglandins; dietary stimulation, e.g. optimizing carbohydrate intake and protein level, optimization of lipid metabolism and amino acid supplementation; light cycle manipulation; and udder stimulation and massage.

Such means have not, generally, met with commercial success. Thus, hormone therapy suffers from non-specificity of results, meager and short term results, adverse side effects and health problems. Dietary manipulation affords inconsistent, short term and, usually, meager results. Light cycle manipulation also affords only inconsistent, meager results and is very difficult to implement. Additionally, many of such means are expensive.

Natural bovine growth hormone is a known material and may be obtained from bovine pituitary glands by extraction and purification. It can be obtained in varying degrees of purity but, of course, the higher the purity the more expensive the final product. The use of high purity natural bovine growth hormone is prohibitive from a cost/effect standpoint.

Thus, the use of natural bovine growth hormone to increase milk production of cows suffers serious disadvantages in that natural bovine growth hormone is very expensive, is obtainable only in limited quantities and contains many byproducts of the pituitary glands from which it is isolated. These byproducts or impurities make administration of natural bovine growth hormone disadvantageous in that relatively large quantities of material must be administered to the animal in order to afford a relatively small dose of the growth hormone.

Bovine growth hormone has a molecular weight of approximately 21,000 in a nonaggregated state, and is produced in the anterior lobe of the pituitary gland. The _in vivo_ production of natural growth hormone is controlled by a complex interaction of stimulatory and inhibitory neural influences and the difficulty of increasing its production and release _in vivo_ is apparent from the following: The ventromedial nucleus of the pituitary contains a stimulatory area for growth hormone control, whereas the preoptic area has been suggested to have an inhibitory function. Somatostatin, a release inhibitory peptide, has been detected in peptidergic neurons and has been demonstrated to inhibit the release of growth hormone both _in vivo_ and _in vitro_. No true growth hormone releasing hormones have yet been isolated; however, synthetic peptides have been demonstrated to release growth hormone _in vitro_ suggesting that a growth hormone releasing factor is probable. Dopamine and norepinephrine are considered to be the most important neurotransmitters in the regulation of growth hormone releasing and inhibiting peptides. Serotonin has been implicated in release of growth hormone during sleep and is correlated to the inhibition produced by non-esterified fatty acids. Amino acid stimulation of growth hormone release may be mediated by histamine. Estrogens, androgens and thyroid hormones can stimulate the release of growth hormone whereas glucocorticosteroids, growth hormone and somatomedins may inhibit release. Evidence also exists that $\beta$-adrenergic agonists inhibit the release of growth hormone and $\alpha$-adrenergic agonists induce the release of growth hormone.

In high-producing dairy cows, a major portion of glucose turnover (60-85%) is utilized by the mammary gland for lactose synthesis. The galactopoetic effects of natural growth hormone are observed as a total increase in milk production and milk fat. During growth hormone administration, lactose synthesis, which is the primary stimulator of milk production, is increased by as much as 10 to 15%. A detailed understanding of how exogenous growth hormone improves milk production and milk quality is unresolved; however, two primary events have to be postulated to occur as either direct or indirect manifestations of exogenous growth hormone administration. The mammary gland must increase its ability to synthesize milk either through an increased proliferation of myoepithelial cells or by increased biosynthetic capacity of those cells pre-existing. Secondly, to facilitate the increased glucose requirement of the mammary gland, growth hormone increases the mobilization of fatty acids from adipose tissues and increases the rate of gluconeogenesis in the liver.

Unlike natural bovine growth hormone, biosynthetically produced growth hormone, e.g. growth hormone produced via recombinant DNA technology, is characterized by an n-terminal methionyl group and freedom from other high molecular weight material which is not usually removed during extraction and purification of bovine pituitary glands. A procedure for the preparation of biosynthetic growth hormone is described in British Patent 1,565,190, issued to the University of California.

As used herein, the term biosynthetic bovine growth hormone (BBGH) includes such hormone which is prepared by any means other than isolation from bovine pituitary gland. Thus, BBGH includes, but is not limited to, that which is prepared by recombinant DNA techniques, by chemical, e.g. solid support, synthesis and by cell free synthesis, e.g. in vitro synthesis.

## Summary of the Invention

This invention relates to the use of BBGH to increase milk production in cows.

In accordance with the present invention, it has been found that the administration of BBGH to cows increases milk production beyond normal and beyond that which is afforded by the administration of natural bovine growth hormone.

Accordingly, the objective of the present invention is to increase the milk production of cows beyond that which is presently available by administration of natural growth hormone or other means, by administering to the cows BBGH.

## Specific Embodiments of the Invention

BBGH has the following known primary amino acid sequence:

$NH_2$-Met-ala-phe-pro-ala-met-ser-leu-ser-gly-leu-phe-ala-asn-ala-val-leu-arg-ala-gln-his-leu-his-gln-leu-ala-ala-asp-thr-phe-lys-glu-phe-glu-arg-thr-tyr-ile-pro-glu-gly-gln-arg-tyr-ser-ile-gln-asn-thr-gln-val-ala-phe-cys-phe-ser-glu-thr-ile-pro-ala-pro-thr-gly-lys-asn-glu-ala-gln-gln-lys-ser-asp-leu-glu-leu-leu-arg-ile-ser-leu-leu-leu-

ile-gln-ser-trp-leu-gly-pro-leu-gln-phe-leu-ser-arg-val-
phe-thr-asn-ser-leu-val-phe-gly-thr-ser-asp-arg-val-tyr-
glu-lys-leu-lys-asp-leu-glu-glu-gly-ile-leu-ala-leu-met-
arg-glu-leu-glu-asp-gly-thr-pro-arg-ala-gly-gln-ile-leu-
lys-gln-thr-tyr-asp-lys-phe-asp-thr-asn-met-arg-ser-asp-
asp-ala-leu-leu-lys-asn-tyr-gly-leu-leu-ser-cys-phe-arg-
lys-asp-leu-his-lys-thr-glu-thr-tyr-leu-arg-val-met-lys-
cys-arg-arg-phe-gly-glu-ala-ser-cys-ala-phe-OH.

In the above formula, any L-amino acid may be substituted for a D-isomer thereof and various amino acids may be interchanged without affecting the activity of the molecule itself. Thus, (1) alanine, leucine, isoleucine, valine and proline are interchangeable, (2) phenylalanine and tryptophan are interchangeable, (3) serine, threonine and tyrosine are interchangeable, (4) asparagine and glutamine are interchangeable, (5) lysine, arginine, histidine and ornithine are interchangeable and (6) aspartic acid and glutamic acid are interchangeable.

Additionally, it is considered that there may be only certain fragments of the entire sequence, together with their spacing and interrelationship with other fragments which are primarily responsible for increasing milk production. Thus, except for those "critical" fragments which are primarily responsible for increased milk production, a further interchange of amino acids or other materials in the BBGH sequence is acceptable. It is therefore acceptable that acids of groups (1) through (6) above may be interchanged between groups in noncritical areas or fragments of the BBGH sequence. Further, it is acceptable to interchange any chemical for any acid or sequence of acids in noncritical fragments of the BBGH sequence as long as the desired activity is not adversely affected.

As stated above, it is not known whether the entire growth hormone molecule is necessary to increase

-6-

milk production or whether a fragment of such molecule or combinations of fragments are responsible for such increase. Further, it is considered that analogs of BBGH and one or more fragments of such analogs or analogs of such fragments may also increase milk production in dairy cows. Accordingly, the present invention contemplates that the administration of BBGH, one or more fragments thereof used separately or in combination and analogs of BBGH, including one or more fragments of such analogs or analogs of fragments used separately or in combination may be utilized to increase milk production in        cows.

It is considered herein that an analog, as described above, may be defined as any change in the primary BBGH sequence or fragments thereof. The change may be as simple as a rearrangement of two acids or as complicated as a rearrangement of multiple substituted fragments and may include spatial considerations. Analogs may also include the substitution  of organic or inorganic molecules in portions or fragments of the sequence. For example, porcine, caprine, ovine and human growth hormones are analogs of BBGH.

The preparation of biosynthetic growth hormone is set forth in detail in British Patent 1,565,190, referred to above and herein incorporated by reference. Using the general method of the cited patent, particularly Example 8 detailing the isolation and purification of growth hormone, one may isolate or chemically synthesize the nucleotide sequence coding for bovine growth hormone and transfer the genetic information contained therein to a microorganism where it may be replicated indefinitely.

Additionally the preparation of BBGH by the cloning of bovine growth hormone is disclosed by Miller et al, "Journal of Bio. Chemistry", Vol. 255, No. 16, pp. 7521-7524 (1980), herein incorporated by reference. Further, the cloning of bovine growth hormone and its expression in bacteria is disclosed by Keshet et al, "Nucleic Acids Research", Vol. 9, No. 1 (1981), incorporated herein by reference.

BBGH may be administered to cows by any method which is effective in delivering the required dosage to the animal's circulatory system. Accordingly, the hormone may be injected, infused or implanted in polymers or other media known for such purpose or administered by any other convenient means in pharmaceutically acceptable base formulations such as solutions, emulsions or gels which may or may not be encapsulated and which may be designed to maximize or regulate the physiological effect of the hormone to improve absorption, improve stability, alter efficacy or alter release patterns or rates. For example, administration may be oral, i.e. via tablet or capsule, intramuscular, intraperitoneal, subcutaneous, intravenous and may include the use of controlled release techniques and may employ the use of infusion pump systems which may be internal or external. Site selection will be dependent on the means used and the effect desired, among other considerations.

BBGH may be administered in dosages of from about 0.005 to about 200,000 micrograms per animal per day. Such a dosage affords milk production that exceeds that obtained with natural bovine growth hormone even though both natural bovine growth hormone and BBGH exhibit similar activity based on bioassay techniques. It is considered that other effects produced by exogenous bovine growth hormone administration will be further enhanced by BBGH administration, e.g. increased meat production by pigs and other farm animals, as well as increased egg, wool and fur production.

Within the range of 0.005 to 200,000 micro-

-8-

grams set forth above, specific dosage ranges will be dependent on a number of variables including the specific animal, its size and its general health and nutritional status. Thus, desirable dosage ranges may be from about 0.05 to about 100,000 micrograms, from about 0.5 to about 50,000 micrograms, from about 5 to about 25,000 micrograms, from about 50 to about 15,000 micrograms and from about 500 to about 5,000 micrograms. Preferably, the dosage would be from about 0.05 to about 50,000 micrograms.

Administration of BBGH may facilitate an increase in other aspects of animal health beyond milk production in bovine, e.g. increased growth. Further, it is possible that milk production of an animal may be controlled by factors other than BBGH, such as somatomedins, enkephalins, endorphins and the like.

It should be understood that the present invention also includes the combination of BBGH with other materials which may affect an animal's growth, general health and productivity. For example, BBGH may be administered concurrently or sequentially in combination with thyroxin, diethylstilbestrol, melangestrol acetate, endocrine and paracrine hormones, antibiotics, coccidiostats, anthelmintics and other therapeutic agents, blood flow controllers, feed intake stimulants and substances which enhance or prolong stability.

The following example represents a preferred embodiment of the invention. It is understood that the invention is not limited thereto.

### Example

Twelve Holstein dairy cows of the same general physical characteristics were selected. Four cows were given daily subcutaneous injections of 25,000 micrograms of natural bovine growth hormone, four were injected with 25,000 micrograms of BBGH and four were injected with 25,000 micrograms of excipient only. Each of the cows were injected once per day for six days. Both the natural bovine growth hormone and BBGH had approximately identical biological activity.

Milk production was increased over normal levels by both the natural bovine growth hormone and BBGH. Normal levels were not increased in the animals receiving the excipient. In the cows receiving natural bovine growth hormone, a 12.2% increase in milk production was observed. In the cows receiving BBGH, a 14% increase in milk production was observed. Based on multiple regression analysis, the responses observed between the use of natural growth hormone and BBGH were significantly different and parallel based on a probability of 95%.

As stated previously, other methods of administration may be used in the above example to control the duration of activity over a period of from about 5 to about 300 days. The use of other dosage levels, as set forth previously, would be expected to afford similar results.

-10-

## CLAIMS

1. Method of increasing milk production in cows comprising administering to said cows a member selected from the group consisting of (a) BBGH, (b) a fragment or fragments of BBGH, and (c) a combination thereof, in an amount effective for increasing said production.

2. Method of Claim 1 wherein said member is biosynthetic bovine growth hormone prepared by a recombinant DNA technique.

3. Method of Claim 2 wherein said member is a fragment or fragments of said hormone.

4. Method of Claim 1 wherein said amount is from about 0.05 to about 100,000 micrograms per animal per day.

5. Method of Claim 4 wherein said amount is from about 5 to about 25,000 micrograms per animal per day.

6. Method of Claim 5 wherein said amount is from about 500 to about 5,000 micrograms per animal per day.

7. Method of Claim 1 wherein said BBGH has a molecular weight of approximately 21,000.

8. Method of Claim 1 wherein said BBGH contains a sequence of 192 amino acids.

9. Method of Claim 1 wherein said amount is effective to increase said milk production over the increase observed using the same amount of natural bovine growth hormone.

10. Method of increasing milk production in dairy cows comprising injecting BBGH at a dosage of from about 0.005 to about 200,000 micrograms per animal per day.

0085036

European Patent
Office

**EUROPEAN SEARCH REPORT**

Application number

EP 83 87 0003

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| D,X | NUCLEIC ACIDS RESEARCH, vol. 9, no. 1, 1981, pages 19-30; E.KESHET et al.: "Cloning of bovine growth hormone gene and its expression in bacteria" *Page 19, "Introduction", paragraph 1* | 1-7,9, 10 | A 61 K 37/36 |
| | --- | | |
| A | CHEMICAL ABSTRACTS, vol. 95, no. 19, 9th November 1981, page 143, no. 162790w, Columbus Ohio (USA); C.J.PEEL et al.: "Effects of exogenous growth hormone on lactational performance in high yielding dairy cows" & J. NUTR. 1981, 111(9), 1662-71.*Abstract* | 1-7,9, 10 | |
| | --- | | |
| P,X | EP-A-0 068 646 (THE UPJOHN COMPANY) *The abstract; page 2, lines 3 and 4; claims* | 1-7,9, 10 | **TECHNICAL FIELDS SEARCHED (Int. Cl. ³)** A 61 K C 12 N |
| | ----- | | |

The present search report has been drawn up for all claims

| Place of search THE HAGUE | Date of completion of the search 22-04-1983 | Examiner RYCKEBOSCH A.O.A. |
|---|---|---|